# DEMANDE DE BREVET EUROPEEN

(11) **EP 1 336 404 A1**
(43) Date de publication de la demande: **20.08.2003**
(21) Numéro de dépôt: 03352003.2
(22) Date de dépôt: 13.02.2003
(51) Int. Cl.: A61K 9/06, A61K 9/107, A61K 9/00

(54) **Composition ophtalmique sous forme d'émulsion**

(30) Priorité: 14.02.2002 FR 0201816
(71) Demandeur: Laboratoires Veyron & Froment, 13000 Marseille (FR)
(72) Inventeur: Dubois, Jacques, 11 100 Narbonne (FR)
(74) Mandataire: Morelle, Guy

(57) **Abrégé**

La présente invention concerne une composition ophtalmique sous forme d'émulsion fluide, comprenant
- une phase aqueuse contenant au moins un agent de viscosité hydrosoluble,
- une phase lipidique contenant au moins un agent de viscosité à une concentration comprise entre 1 et 6 % en poids de ladite phase lipidique, ladite phase lipidique constituant 1,5 à 13 % en poids de ladite composition,
et en ce que ladite composition ne contient pas d'agent tensioactif.
Ladite composition peut en outre contenir au moins un principe actif et un ou plusicurs additifs. Elle peut être utilisée pour la préparation de produits destinés aux soins de la sphère oculaire ou au traitement des affections ophtalmiques. Un procédé de préparation de doses stériles est également revendiqué dans lequel la composition est conditionnée en flacons unitaires avant d'être stérilisée en autoclave durant 20 minutes à la température de 120 °C.

## Description

La présente invention se situe dans le domaine de l'ophtalmologie, et plus particulièrement dans le domaine des préparations destinées à améliorer le confort ou à apporter des substances médicamenteuses à l'oeil lui-même ainsi qu'à la paupière.

Les produits ophtalmiques actuellement disponibles dans le commerce permettent de soulager et de traiter une large gamme d'affections: sécheresse oculaire, conjonctivites, infections, inflammations, etc..... Certains peuvent être considérés comme des produits de confort agissant en premier lieu sur des manifestations symptomatiques, tels les produits lubrifiants destinés à compenser la sécheresse oculaire, appelés parfois larmes artificielles, d'autres sont des médicaments, composés principalement d'un véhicule et d'au moins un principe actif destiné à traiter un affection particulière. Dans tous les cas, ils doivent être stériles.

Ils sont présentés sous deux formes différentes: liquide ou pâteuse. Sous la forme liquide, sont préparées soit des solutions aqueuses, soit des suspensions, ou encore aussi parfois des gels fluides dont le véhicule est l'eau distillée, et qui sont destinés à être instillés dans le sac conjonctival. Sous la forme pâteuse, sont préparées des pommades anhydres dont le véhicule est essentiellement composé d'hydrocarbures paraffiniques auxquels peut être adjointe ou non de la lanoline. Ces pommades sont généralement utilisées en application sur la conjonctive, sur l'oeil ou sous la paupière.

Cependant ces produits présentent un certain nombre d'inconvénients, liés en partie au fait que l'oeil est un organe sensible dont la surface doit toujours rester hydratée et lubrifiée. D'ordinaire, le film lacrymal apporte l'humidité nécessaire en même temps qu'une composante lipidique protectrice sécrétée par les glandes de Meibomius. Or les deux formes galéniques précitées répondent à l'une ou à l'autre de ces exigences, sans réussir à les concilier.

Les formes pâteuses, bien adaptées pour une application sur la paupière externe, sont d'emploi peu attractif dès lors qu'il s'agit d'étaler une dose de crème de viscosité élevée sur le globe oculaire. Au demeurant la difficulté d'étalement sur l'oeil réduisant leur efficacité et la gêne visuelle en résultant, limitent l'emploi de telles formes pâteuses en tant que produits ophtalmiques.

Les formes liquides, appelées collyres, présentées en général en gouttes, ont l'avantage d'avoir une viscosité réduite. Si elles apportent une bonne humidification de la conjonctive au moment de leur instillation dans l'oeil, en revanche, elles sont rapidement éliminées et possèdent un pouvoir lubrifiant très faible. En outre, les gouttes ophtalmiques liquides sont des phases aqueuses, dont l'instillation dans l'oeil induit un dysconfort se manifestant par des sensations de rugosité et de gêne sous les paupières.

Pour surmonter ce problème, on a cherché à incorporer des substances grasses en dispersion dans la phase aqueuse, en les stabilisant à l'aide par exemple de composés tensioactifs. Cependant l'agressivité de ces derniers vis-à-vis des milieux biologiques limite fortement leur emploi au niveau de l'oeil. On a aussi essayé d'utiliser des composés lipidiques ayant une solubilité dans l'eau suffisante pour former un produit homogène et stable aux concentrations désirées sans avoir recours à des composés tensioactifs. Certains lubrifiants ophtalmiques liquides contiennent, par exemple, des paraffines liquides. Mais leur instillation dans l'oeil trouble la vision et induit une telle gêne chez le patient que leur emploi n'est envisageable qu'au moment du coucher.

Ainsi, il apparaît qu'aucun des produits actuellement utilisés en ophtalmologie ne donne entièrement satisfaction. La présente invention propose une solution nouvelle, consistant en une composition destinée à un usage ophtalmique se présentant sous la forme d'une émulsion stable sans ajout de tensioactif, et permettant de préparer un collyre dont les caractéristiques répondent aux critères essentiels de compatibilité avec l'oeil, de confort et d'efficacité, grâce en particulier à leur action reconstituante du film lacrymal.

Jusqu'à présent, il n'existe pas de composition ophtalmique sous forme d'émulsion susceptible d'être instillée dans le sac conjonctival. Cette situation s'explique par le fait qu'il est généralement admis que les émulsions contiennent nécessairement des agents tensioactifs et des émulgateurs. Leur présence entraîne, en raison de leur effet sur la tension superficielle des liquides, une irritation locale. La réaction d'intolérance qui se manifeste alors est beaucoup plus marquée lorsque le site d'application est une muqueuse, telle que l'oeil ou la paupière interne, que lorsque le produit est destiné à une application cutanée.

On souligne, à l'appui de cette remarque que les émulsions destinées aux soins du visage doivent être appliquées à distance de l'oeil pour éviter tout risque de réaction. En cas d'incident, il est aussi conseillé d'éliminer l'émulsion par un lavage prolongé de l'oeil et des paupières.

Le caractère innovant de la composition faisant l'objet de la présente invention réside dans la réalisation d'une formule d'émulsion applicable sur l'oeil, sans provoquer de réaction d'intolérance. Cet objectif est atteint sans l'aide d'agent tensioactif émulgateur, ce qui va à l'encontre des connaissances admises habituellement.

Ce résultat inattendu a pu être obtenu en ajustant la viscosité de la phase aqueuse d'une part et celle de la phase lipidique d'autre part, de façon à obtenir une consistance adéquate du mélange pour stabiliser cette composition.

La présente invention concerne donc une nouvelle présentation de collyre sous forme d'émulsion, et son procédé d'obtention. Cette présentation se distingue des produits ophtalmiques existants par le fait qu'elle s'apparente à la forme émulsion et qu'elle présente un tolérance locale ― oculaire notamment ― parfaite.

Plus précisément, la présente invention concerne une composition destinée à un usage ophtalmique se présentant sous la forme d'une émulsion, comprenant:
- a) une phase aqueuse contenant au moins un agent de viscosité hydrosoluble,
- b) une phase lipidique contenant au moins un agent de viscosité à une concentration comprise entre 1 et 6 % en poids de ladite phase lipidique, ladite phase lipidique constituant 1,5 à 13 % en poids de ladite composition,
et en ce que ladite composition ne contient pas d'agent tensioactif.

La composition ainsi obtenue peut être employée telle quelle, en tant que produit humectant et lubrifiant de l'oeil. Dans ce cas, elle peut alors faire fonction de larmes artificielles utilisées par exemple, dans le traitement du syndrome de l'oeil sec.

La composition selon l'invention peut aussi constituer le support d'au moins un principe actif, tout particulièrement un principe actif destiné au traitement d'une affection ophtalmique. Par exemple, la composition selon l'invention peut contenir, outre les ingrédients définis ci-dessus, un principe actif à usage ophtalmique choisi parmi les composés suivants: antiseptique, antibiotique, anti-inflammatoire, anti-allergique, antiglaucomateux, vaso-constricteur, mydryatique, myotique. Un principe actif peut être introduit dans une des deux phases de la composition, ou bien dans les deux, selon sa solubilité et sa compatibilité avec l'une et l'autre phase.

L'agent de viscosité de la phase aqueuse est choisi en fonction de sa bonne tolérance oculaire parmi les épaississants et viscosants classiquement employés par l'homme du métier. On citera par exemple la gélose, la gomme, les alginates, la cellulose et ses dérivés, les polymères acryliques. Notre préférence va vers deux types de viscosants, qui présentent une tolérance oculaire irréprochable:
- les dérivés cellulosiques, notamment la méthylcellulose, l'éthylcellulose, l'hydroxy-propylcellulose, la carboxyméthylcellulose;
- les polymères acryliques ou carbomères, toujours neutralisés par un alcalin minéral (tel que la soude ou la potasse), ou organique (tel que la triéthanolamine ou la diisopropylamine).

La teneur de l'agent viscosant dans la phase aqueuse est variable selon la nature dudit agent et selon son pouvoir viscosant. Par exemple, si le polymère carboxyvinylique est choisi comme agent viscosant, il sera avantageusement introduit à raison de 0,15 à 1,5 % de la phase aqueuse, tandis que l'hydroxypropyléthylcellulose peut être employée à des teneurs comprises entre 1 et 3 % de la phase aqueuse, en poids. De manière générale, la consistance de la phase aqueuse doit être comparable à celle de la phase lipidique. L'homme du métier saura adapter les concentrations de ces produits bien connus sans difficulté.

L'agent de viscosité (ou agent de consistance) présent dans la phase lipidique est une substance semi-solide à température ambiante, dont le point de fusion est supérieur à 45°C. Il est facilement solubilisé à chaud dans la phase grasse, puis en refroidissant, il retrouve sa consistance semi-solide et confère à la phase lipidique une consistance qui est réglée par sa teneur dans le mélange obtenu.

Un tel agent viscosant est choisi parmi les composés ayant un point de fusion supérieur à 45°C appartenant aux groupes chimiques suivants: les hydrocarbures saturés, les graisses et cires d'origine végétale, animale, minérale ou synthétique, les triglycérides, les diglycérides et les monoglycérides. Le choix de la phase grasse est très ouvert. On peut citer entre autres substances répondant aux critères définis dans la cadre de la présente invention: les huiles végétales, les huiles minérales, les huiles de silicone, le perhydrosqualène, le palmitate d'isopropyle, et d'autres encore que l'homme du métier connaît bien.

Rappelons que la caractéristique décisive de cet ingrédient réside dans sa neutralité et sa bonne tolérance vis-à-vis des structures histologiques particulièrement sensibles que sont l'oeil et la paupière. Quelle que soit la substance retenue, elle ne doit pas dépasser une certaine concentration, et plus précisément l'agent de viscosité de la phase lipidique de la composition selon l'invention est présent à une concentration comprise entre 1 et 6 % en poids de ladite phase lipidique totale.

La composition selon l'invention peut en outre contenir un ou plusieurs additifs, à la condition expresse qu'ils soient compatibles avec un usage ophtalmique. Par exemple, un tel additif peut être choisi parmi: un parfum, un agent conservateur, un anti-oxydant, un tampon, un isotonisant, un chélateur. Ils seront introduits au choix, dans une des phases au cours de leur préparation, ou bien après le mélange de celles-ci, selon les conditions les plus adaptées connues de l'homme du métier, avec le soucis constant de la bonne tolérance du produit final.

Comme expliqué plus haut, la composition selon l'invention a pu être obtenue sous forme d'émulsion en ajustant la viscosité de la phase aqueuse d'une part et celle de la phase lipidique d'autre part, de façon à obtenir une consistance de ladite composition telle qu'elle ne nécessite pas l'adjonction de tensioactif. Cette exigence étant remplie grâce au choix judicieux des agents viscosants comme décrits précédemment.

Il reste à mélanger les deux phases aqueuses et lipidiques de manière à obtenir en fin de compte un collyre comparable au film lacrymal naturel, dont il doit reconstituer la composition. La proportion adéquate des deux phases peut être définie par la part de la phase lipidique entrant dans la composition finale. Selon la présente invention la phase lipidique constitue de 1,5 à 13 % en poids de la composition complète. Ainsi, le collyre-émulsion selon l'invention a une composition telle qu'il mime le film lacrymal dans ses caractéristiques chimiques et physiques, ce qui permet outre une tolérance parfaite, une instillation dans l'oeil sans troubler la vue, autorisant son usage en toutes circonstances.

Les collyre-émulsions selon l'invention peuvent être préparés industriellement de différentes manières. Ils devront toujours être parfaitement stériles, quels que soient le mode de préparation et le conditionnement choisis.

Une méthode repose sur la technique de filtration stérilisante. Selon cette méthode, chaque phase est d'abord préparée séparément. Dans un réacteur à 70 °C, l'agent viscosant choisi est ajouté à la base lipidique, et malaxé jusqu'à obtention d'une masse homogène fluide. Par ailleurs, la base aqueuse constituée d'eau purifiée et d'éventuels additifs hydrosolubles, est portée dans un réacteur à 70 °C. Puis, la phase lipidique et la base aqueuse sont soumises à une étape de filtration stérilisante par passage sur une ou plusieurs membranes de faible porosité (0,22 à 0,1 µm). L'agent viscosant de la phase aqueuse dans ce cas, ne doit pas être introduit avant filtration sous peine d'obturation des filtres.

Les opérations suivantes se déroulent obligatoirement en conditions stériles. La phase lipidique est introduite dans la phase aqueuse sous forte agitation, en même temps que l'agent viscosant de la phase aqueuse préalablement stérilisé, par un sas stérile. Le tout est refroidi progressivement jusqu'à 25 °C, en maintenant une agitation douce. La composition obtenue est stable, et peut être répartie dans le conditionnement désiré (flacon styligoutte, ampoule-bouteille en matière plastique, ou autre), en salle stérile.

Cette méthode est utilisée quand l'actif (éventuel) est thermolabile. L'actif peut être introduit dans le réacteur par le sas stérile durant le refroidissement, dès que la température le permet.

Une seconde méthode met en valeur l'intérêt de l'invention en évitant toute cette chaîne de fabrication très lourde sur le plan industriel. Elle consiste à préparer la composition dans les conditions classiques, par mélange des phases préalablement préparées sans contrainte de stérilité particulière, puis à conditionner en flacons unitaires (flacons styligouttes, ampoules-bouteilles en matière plastique, ou autres) et enfin à réaliser une stérilisation des conditionnements ainsi remplis. Pour effectuer la stérilisation, on met à profit une caractéristique particulièrement avantageuse de la présente invention, à savoir la bonne stabilité thermique de la composition revendiquée.

En effet celle-ci se prête à une stérilisation à l'autoclave. On pourra alors procéder à une stérilisation de la composition selon l'invention en autoclave durant 20 minutes à la température de 120 °C. Ce procédé de fabrication constitue un avantage considérable en terme de coût de revient industriel et constitue un objet de la présente invention.

La présente invention a ainsi également pour objet un procédé de préparation d'une composition ophtalmique telle que décrite précédemment, comprenant au moins les étapes consistant à
a) préparer une phase lipidique en introduisant dans un réacteur au moins un corps gras et un agent de viscosité à une concentration comprise entre 1 et 6 % en poids de la phase lipidique, et en chauffant à une température voisine de 70°C, sous agitation, jusqu'à obtention d'une phase homogène,
b) préparer une phase aqueuse en introduisant dans un réacteur au moins un agent de viscosité et de l'eau et en chauffant à une température voisine de 70°C, sous agitation, jusqu'à obtention d'une solution homogène,
c) introduire la phase lipidique dans la phase aqueuse sous forte agitation,
d) laisser refroidir le mélange obtenu sous faible agitation jusqu'à 25°C,
e) répartir le mélange obtenu en conditionnements unitaires,
f) stériliser les conditionnements ainsi remplis dans un autoclave à une température de 120 °C durant 20 minutes.

Selon une variante de mise en oeuvre du procédé précédent, on peut ajouter en outre durant une des étapes a) à e) au moins l'un des composés suivants: un principe actif à usage ophtalmique, un parfum, un agent conservateur.

Cette composition ophtalmique, qu'on peut qualifier de collyre-émulsion, se prête à de nombreuses applications, en raison de la présence d'une phase lipidique représentant au plus 13 % de la formule.

Elle peut être avantageusement utilisée pour la préparation de produits destinés aux soins de la sphère oculaire, par exemple comme lubrifiant de l'oeil chez les patients souffrant du syndrome de l'oeil sec. Son activité est bien supérieure à celle des larmes artificielles ou des gels ophtalmiques existants, car la présentation sous forme d'émulsion permet d'une part d'humidifier la conjonctive, et d'autre part de reconstituer un film lacrymal complet, assurant ainsi un confort exceptionnel pour l'utilisateur, et prolongeant la durée d'efficacité du produit, ce qui permet une fréquence d'utilisation moins élevée.

Elle peut être utilisée avec les mêmes avantages, à la préparation d'un produit destiné au traitement des affections ophtalmiques en y incorporant en outre un principe actif doté de l'effet thérapeutique recherché.

Les exemples suivants permettront de mieux comprendre l'invention.

### EXEMPLE 1

### Collyre-émulsion lubrifiant

Un collyre-émulsion destiné à la lubrification de la conjonctive chez les patients soufrant du syndrome de l'oeil sec est préparé selon un des procédés décrits plus haut, avec les ingrédients suivants donnés pour une composition centésimale:

| • Phase lipidique: | |
|---|---|
| - Monostéarate de glycérol | 0,35 g |
| - Huile de paraffine | 5,65 g |

| • Phase aqueuse: | |
|---|---|
| - Polymère carboxyvinylique | 0,50 g |
| - Bicarbonate de sodium | 0,75 g |
| - Sorbitol | 0,25 g |
| - Eau distillée d'Hamamélis | 10,00 g |
| - Eau purifiée | QSP 100 g |

Cette formule présente un pH de 7,2. Elle est isotonique des larmes.
Elles est avantageusement conditionnée en récipients unidoses de 0,50 ml.

### EXEMPLE 2

### Collyre-émulsion anti-allergique

Un collyre-émulsion anti-allergique contenant du cromoglycate de sodium, est préparé selon un des procédés décrits plus haut, avec les ingrédients suivants donnés pour une composition centésimale:

| • Phase lipidique: | |
|---|---|
| - Monostéarate de glycérol | 0,60 g |
| - Huile végétale | 11,40 g |

| • Phase aqueuse: | |
|---|---|
| - Hydroxypropyléthylcellulose | 1,80 g |
| - Cromoglycate de sodium | 2,00 g |
| - Edétate de sodium | 0,10 g |
| - Chlorure de benzalkonium | 0,01 g |
| - Eau purifiée | QSP 100 g |

Cette préparation peut être conditionnée en flacon styligoutte ou en tube-canule de 5 ml et appliquée dans l'oeil et sur les paupières. Elle doit être utilisée dans les 15 jours suivant l'ouverture du conditionnement.

### EXEMPLE 3

### Collyre-émulsion antibiotique

Un collyre-émulsion antibiotique contenant de l'acide fusidique, est préparé selon un des procédés décrits plus haut, avec les ingrédients suivants donnés pour une composition centésimale:

| • Phase lipidique: | |
|---|---|
| - Monostéarate de glycérol | 0,50 g |
| - Huile de paraffine | 11,00 g |
| - Acide fusidique (sel sodique) | 1,00 g |

| • Phase aqueuse: | |
|---|---|
| - Polymère carboxyvinylique | 0,35 g |
| - Edétate de sodium | 0,01 g |
| - Mannitol | 2,00 g |
| - Chlorure de benzalkonium | 0,01 g |
| - Solution d'hydroxyde de sodium | QSP pour pH voisin de 5,6 |
| - Eau purifiée | QSP 100 g |

Cette préparation peut être conditionnée en tube de 3 g. Elle est appliquée dans l'oeil et sur les paupières. Elle doit être utilisée dans les 15 jours suivant l'ouverture du tube.

## Revendications

1. Composition lubrifiante fluide mimant le film lacrimal se présentant sous la forme d'une émulsion, ***caractérisée en ce qu***'elle comprend
- a) une phase aqueuse contenant au moins un agent de viscosité hydrosoluble,
- b) une phase lipidique contenant au moins un agent de viscosité à une concentration comprise entre 1 et 6 % en poids de ladite phase lipidique, ladite phase lipidique constituant 1,5 à 13 % en poids de ladite composition,
et en ce que ladite composition ne contient pas d'agent tensioactif.

2. Composition selon la revendication 1, ***caractérisée en ce qu***'elle comprend en outre un principe actif à usage ophtalmique.

3. Composition selon la revendication 2, ***caractérisée en ce que*** ledit principe actif à usage ophtalmique est choisi parmi les composés suivants: antiseptique, antibiotique, anti-inflammatoire, anti-allergique, antiglaucomateux, vaso-constricteur, mydryatique, myotique.

4. Composition selon une des revendications précédentes, ***caractérisée en ce qu***'au moins un agent de viscosité contenu dans la phase aqueuse est choisi parmi les composés suivants: la gélose, la gomme, les alginates, la cellulose et dérivés cellulosiques, les polymères acryliques, et de préférence parmi la méthylcellulose, l'éthylcellulose, l'hydroxypropyl-cellulose, la carboxyméthylcellulose.

5. Composition selon une des revendications précédentes, ***caractérisée en ce qu***'au moins un agent de viscosité contenu dans la phase lipidique est choisi parmi les composés ayant un point de fusion supérieur à 45°C suivants: les hydrocarbures saturés; les graisses et cires d'origine végétale, animale, minérale ou synthétique; les triglycérides; les mono- et diglycérides.

6. Composition selon l'une des revendications précédentes, ***caractérisée en ce qu***'elle comprend en outre un additif compatible avec un usage ophtalmique choisi parmi: un parfum, un agent conservateur, un anti-oxydant, un tampon, un isotonisant, un chélateur.

7. Procédé de préparation d'une composition selon la revendication 1, comprenant au moins les étapes consistant à:
a) préparer une phase lipidique en introduisant dans un réacteur au moins un corps gras et un agent de viscosité à une concentration comprise entre 1 et 6 % en poids de la phase lipidique, et en chauffant à une température voisine de 70°C, sous agitation, jusqu'à obtention d'une phase homogène,
b) préparer une phase aqueuse en introduisant dans un réacteur au moins un agent de viscosité et de l'eau et en chauffant à une température voisine de 70°C, sous agitation, jusqu'à obtention d'une solution homogène,
c) introduire la phase lipidique dans la phase aqueuse sous forte agitation,
d) laisser refroidir le mélange obtenu sous faible agitation jusqu'à 25°C,
e) répartir le mélange obtenu en conditionnements unitaires,
f) stériliser les conditionnements ainsi remplis dans un autoclave à une température de 120 °C durant 20 minutes.

8. Procédé selon la revendication précédente, dans lequel est ajouté en outre durant une des étapes a) à e) au moins l'un des composés suivants: un principe actif à usage ophtalmique, un parfum, un agent conservateur.

9. Composition susceptible d'être obtenue par un procédé selon la revendication 8, ***caractérisée en ce qu***'elle a pour formule:
| • Phase lipidique: | |
|---|---|
| - Monostéarate de glycérol | 0,35 g |
| - Huile de paraffine | 5,65 g |
| • Phase aqueuse: | |
|---|---|
| - Polymère carboxyvinylique | 0,50 g |
| - Bicarbonate de sodium | 0,75 g |
| - Sorbitol | 0,25 g |
| - Eau distillée d'Hamamélis | 10,00 g |
| - Eau purifiée | QSP 100 g |

10. Composition susceptible d'être obtenue par un procédé selon la revendication 8, ***caractérisée en ce qu***'elle a pour formule:
| • Phase lipidique: | |
|---|---|
| - Monostéarate de glycérol | 0,60 g |
| - Huile végétale | 11,40 g |
| • Phase aqueuse: | |
|---|---|
| - Hydroxypropyléthylcellulose | 1,80 g |
| - Edétate de sodium | 0,10 g |
| - Chlorure de benzalkonium | 0,01 g |
| - Principe actif | dose thérapeutique utile |
| - Eau purifiée | QSP 100 g |

11. Composition susceptible d'être obtenue par un procédé selon la revendication 8, **caractérisée en ce qu'**elle a pour formule:
| • Phase lipidique: | |
|---|---|
| - Monostéarate de glycérol | 0,50 g |
| - Huile de paraffine | 11,00 g |
| - Principe actif | dose thérapeutique utile |
| • Phase aqueuse: | |
|---|---|
| - Polymère carboxyvinylique | 0,35 g |
| - Edétate de sodium | 0,01 g |
| - Mannitol | 2,00 g |
| - Chlorure de benzalkonium | 0,01 g |
| - Solution d'hydroxyde de sodium | QSP pour pH voisin de 5,6 |
| - Eau purifiée | QSP 100 g |

12. Application d'une composition selon l'une des revendications 1 à 6 et 9 à 11, à la préparation d'un produit destiné au traitement des affections ophtalmiques ou aux soins de la sphère oculaire.
